(19) Europäisches Patentamt
European Patent Office
Office européen des brevets

(11) **EP 2 361 644 B1**

(12) **EUROPEAN PATENT SPECIFICATION**

(45) Date of publication and mention of the grant of the patent:
**16.04.2014 Bulletin 2014/16**

(51) Int Cl.:
***A61M 1/36*** *(2006.01)*      ***A61M 1/34*** *(2006.01)*

(21) Application number: **09828830.1**

(22) Date of filing: **24.11.2009**

(86) International application number:
**PCT/JP2009/006327**

(87) International publication number:
**WO 2010/061580 (03.06.2010 Gazette 2010/22)**

(54) **UTILIZATION OF ANTIOXIDANT AGENT IN DEVICE FOR SELECTIVELY REMOVING HLA-DR-POSITIVE MONOCYTES AND FOR PRODUCTION OF SAME**

VERWENDUNG EINES ANTIOXIDATIONSMITTELS BEI EINEM GERÄT ZUR SELEKTIVEN ENTFERNUNG VON HLA-DR-POSITIVEN MONOZYTEN UND ZUR HERSTELLUNG DAFÜR

UTILISATION D'UN AGENT ANTIOXYDANT DANS UN DISPOSITIF D'ÉLIMINATION SÉLECTIVE DE MONOCYTES POSITIFS À L'ANTIGÈNE HLA-DR ET POUR UNE PRODUCTION DE CELUI-CI

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.11.2008 JP 2008300176**

(43) Date of publication of application:
**31.08.2011 Bulletin 2011/35**

(73) Proprietor: **Asahi Kasei Medical Co., Ltd.**
**Tokyo (JP)**

(72) Inventors:
• **KASHIO, Yumiko**
**Tokyo 101-8101 (JP)**

• **SAKURAI, Masami**
**Tokyo 101-8101 (JP)**
• **SUKEGAWA, Takeshi**
**Tokyo 101-8101 (JP)**

(74) Representative: **von Kreisler Selting Werner**
**Deichmannhaus am Dom**
**Bahnhofsvorplatz 1**
**50667 Köln (DE)**

(56) References cited:
**EP-A- 1 016 425**     **EP-A1- 1 553 113**
**WO-A1-2004/018085**    **JP-A- 9 075 076**
**JP-A- 11 322 618**      **JP-A- 2005 287 621**
**JP-A- 2005 323 652**    **JP-A- 2007 307 280**
**JP-A- 2008 125 932**

**Description**

Technical Field

The present invention relates to a device for selectively removing HLA-DR-positive monocytes and use of an antioxidant for manufacture of the selective removal device.

Background Art

A technique of selectively removing leukocytes using a leukocyte removal filter has been developed and then improved for the purpose of treating autoimmune diseases such as systemic lupus erythematosus, rheumatoid arthritis, multiple sclerosis, ulcerative colitis and Crohn's disease, leukemia, and other diseases. Leukocyte removal devices using a non-woven fabric or the like as a filter have been currently developed.

Immune reactions caused by various immunocytes are deeply involved in diseases that become the targets of leukocyte removal therapy. A T cell, which plays an important role in immune reaction, starts its activity when an antigen binds to an antigen receptor existing on the surface of the T cell. The presence of an antigen-presenting cell is necessary for the reaction by the T cell. The T cell responds to a combination of a major histocompatibility antigen (MHC antigen) on the antigen-presenting cell and an antigen.

The MHC antigen is referred to as an HLA antigen in the case of a human, and an HLA-DR antigen is expressed in a fraction acting as an antigen-presenting cell in leukocytes. The activated T cell accepts antigen presentation from other leukocyte fractions, and it becomes more active, so that the immune reaction progresses. The activated leukocytes cause damage on intestinal epithelium cells or osteoclasis, which would lead to the development of disease.

Antigen-presenting cells that are HLA-DR antigen-positive cells are thus considered to be important immunocytes, which are deeply involved in an immune reaction from the initial stage thereof and also in the subsequent reactions.

It has been known that patients with rheumatoid arthritis or ulcerative colitis, which are the targets diseases of the leukocyte removal device, have HLA-DR antigen-positive cells in higher proportion than those of healthy individuals (see Non-Patent Documents 1 and 2).

As a technique of selectively removing leukocytes using a leukocyte removal filter, Patent Document 1 discloses a method in which a large amount of citric acid is added to blood to decrease the viscosity of hematopoietic stem cells, as appropriate, so as to improve the recovery rate of the hematopoietic stem cells.

Further, Patent Document 2 discloses a filter device for selectively removing monocytes and/or monocyte-derived macrophages.

Prior Art Documents

Patent Documents

Patent Document 1: Japanese Patent Laid-Open No. 11-322618
Patent Document 2: Japanese Patent No. 3812909

EP 1 553 113 A1 describes a device for selectively removing leucocytes employing a cell removal filter formed by a non-woven fabric in combination with an antioxidant and an anticoagulant. The surface of the cell removal filter is made from surface coated polyester.

Non-Patent Documents

Non-Patent Document 1: Mottonen M. et.al., Immunology, 2000, vol. 100, pp. 238-244
Non-Patent Document 2: Sawada K. et.al., Jpn. J. Apheresis., 1995, vol. 14. No. 1, pp. 48-50

Summary of the Invention

Problem to be Solved by the Invention

Leukocyte fractions other than lymphocytes including antigen-presenting cells and T cells include granulocytes.

Granulocytes are also involved in the development of disease due to cytotoxicity caused by generation of active oxygen. Monocytes that are antigen-presenting cells or lymphocytes activated as a result of antigen presentation are significantly involved in the activation of granulocytes. It is considered that almost all of immune reactions can be regulated by controlling antigen presentation occurring at the initial stage of the immune reaction caused by leukocytes.

The conventional leukocyte removal therapy has only removed various fractions of immunocytes. If antigen-presenting cells deeply involved in immune reactions could be selectively removed, the efficiency of the therapeutic effect of leukocyte removal would be further improved. In particular, in order to increase the efficiency of the therapeutic effect of leukocyte removal, it is considered important to selectively remove HLA-DR-positive monocytes, which incorporate large particulate antigens as a result of phagocytosis and then present such antigens.

However, a device for selectively removing HLA-DR-positive monocytes, which can be used clinically, has not yet been known. Neither Patent Document 1 nor 2 discloses a means to be able to selectively remove HLA-DR-positive monocytes.

Under the aforementioned circumstances, it is an object of the present invention to provide a device for selectively removing HLA-DR-positive monocytes, which is usable in clinically.

Means for Solving the Problem

The invention is defined by the independent claims. As a result of intensive studies directed towards achieving the aforementioned object, the present inventors have found that HLA-DR-positive monocytes can be selectively removed by supplying blood to a removal device comprising a cell removal filter that has been allowed to come into contact with a filling solution comprising an antioxidant and has been then subjected to radiation sterilization, in the presence of an anticoagulant comprising citric acid or a salt thereof, thereby completing the present invention.

Specifically, the present invention provides use of an antioxidant for manufacture of a device for selectively removing HLA-DR-positive monocytes, and a device for selectively removing HLA-DR-positive monocytes, and a method for selectively removing HLA-DR-positive monocytes, as described below.

(1) Use of an antioxidant for manufacture of a device for selectively removing HLA-DR-positive monocytes, which selectively removes HLA-DR-positive monocytes from blood comprising an anticoagulant, wherein
the device for selectively removing HLA-DR-positive monocytes comprises a cell removal filter formed by a surface-unmodified polyester non-woven fabric,
the cell removal filter is filled with a filling solution comprising an antioxidant and is subjected to radiation sterilization,
the anticoagulant is at least one selected from the group consisting of citric acid and a salt thereof, and
the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.
(2) The use of the antioxidant according to (1) above, wherein the filling solution comprises 300 ppm or more of sodium pyrosulfite.
(3) The use of the antioxidant according to (1) or (2) above, wherein the radiation is gamma ray or electron beam.
(4) A device for selectively removing HLA-DR-positive monocytes, which selectively removes HLA-DR-positive monocytes from blood comprising an anticoagulant, wherein
the device for selectively removing HLA-DR-positive monocytes comprises a cell removal filter formed by a surface-unmodified polyester non-woven fabric,
the cell removal filter is filled with a filling solution comprising an antioxidant and is subjected to radiation sterilization,
the anticoagulant is at least one selected from the group consisting of citric acid and a salt thereof, and
the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.
(5) The selective removal device according to (4) above, wherein the filling solution comprises 300 ppm or more of sodium pyrosulfite.
(6) The selective removal device according to (4) or (5) above, wherein the radiation is gamma ray or electron beam.
(7) A device for selectively removing HLA-DR-positive monocytes comprising:

a cell removal filter filled with a filling solution comprising an antioxidant and subjected to radiation sterilization, wherein the cell removal filter is connected with a first flow passage on an upstream side and a second flow passage on a downstream side; and
a vessel containing at least one anticoagulant selected from the group consisting of citric acid and a salt thereof, wherein the vessel is connected with the first flow passage and is capable of supplying blood comprising the anticoagulant to the cell removal filter.

(8) A method for selectively removing HLA-DR-positive monocytes from blood comprising an anticoagulant, using a selective removal device comprising a cell removal filter formed by a surface-unmodified polyester non-woven fabric, comprising steps of:

filling the cell removal filter with a filling solution comprising an antioxidant;
subjecting the cell removal filter filled with the filling solution to radiation sterilization; and
supplying blood that comprises an anticoagulant comprising citric acid or a salt thereof to the filter device and filtrating it, wherein
the anticoagulant is at least one selected from the group consisting of citric acid and a salt thereof, and
the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.

(9) The method according to (8) above, wherein the filling solution comprises 300 ppm or more of sodium pyrosulfite.
(10) The method according to (8) or (9) above, wherein the radiation is gamma ray or electron beam.

Advantageous Effects of the Invention

The present invention can provide a device for selectively removing HLA-DR-positive monocytes, which can be safely used clinically.

Brief Description of Drawings

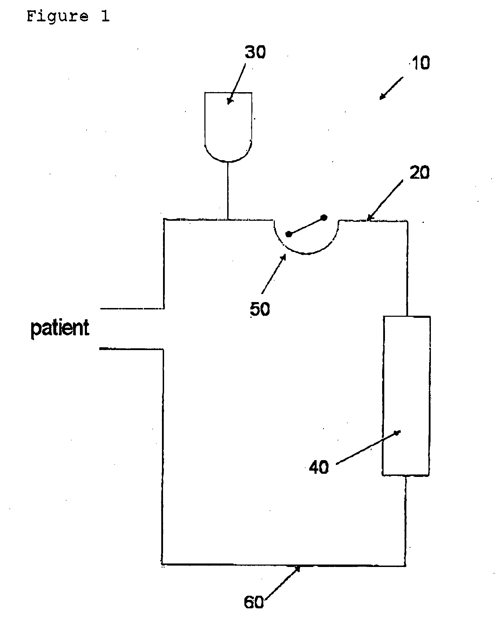

[Figure 1] Figure 1 represents a schematic view showing an example of the selective removing device of the present embodiment.

Mode for Carrying out the Invention

The embodiment for carrying out the present invention (hereinafter referred to as "the present embodiment") will be described in detail below. However, the present invention is not limited to the following embodiment, and it may be modified in various ways and may be carried out within the range of the gist thereof.
The use of an antioxidant for production of a device for selectively removing HLA-DR-positive monocytes of the present embodiment is a use of an antioxidant for production of a device for selectively removing HLA-DR-positive monocytes, which selectively removes HLA-DR-positive monocytes from blood comprising an anticoagulant, wherein the device for selectively removing HLA-DR-positive monocytes comprises a cell removal filter formed by a surface-unmodified polyester non-woven fabric, the cell removal filter is filled with a filling solution comprising an antioxidant and is subjected to radiation sterilization, the anticoagulant is at least one selected from the group consisting of citric acid and a salt thereof, and the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.
The device for selectively removing HLA-DR-positive monocytes of the present embodiment (hereinafter simply referred to as a "selective removal device") has a particularly high rate of removing HLA-DR-positive monocytes, in comparison to conventional leukocyte removal devices. It can be used to selectively remove HLA-DR-positive monocytes from whole blood, plasma, or a blood-derived preparation such as an erythrocyte preparation or a platelet preparation.
The vessel used as a selective removal device is not particularly limited, as long as it is a vessel that can have a cell removal filter.
Examples of the selective removal device include, but are not particularly limited to, a vessel filled with cell removal filters in a laminated state and a vessel filled with a cell removal filter that is cylindrically wound.
Examples of the shape of the vessel used as a selective removal device include, but are not particularly limited to, a cylinder, and prismatic bodies such as a triangular prism, a quadrangular prism, a hexagonal prism and an octagonal prism.
The shape of the vessel used as a selective removal device is preferably a cylinder. A preferred example of a cylindrical selective removal device includes a vessel in which blood flows from the outer circumference of the cylinder into the inside thereof, and in which the blood gathers in the innermost area and then exits from a blood discharge port. Also, there is preferably used a vessel having a shape in which the cross section of the vessel decreases in the direction from the inlet of blood to the outlet thereof.
In the present embodiment, the selective removal device may further comprise a tube, a circuit, and the like, which are used to treat blood or to supply or discharge a liquid for washing the selective removal device.

An example of materials for the vessel used as a selective removal device includes a polycarbonate resin.

The cell removal filter of the present embodiment comprises a surface-unmodified polyester non-woven fabric.

The cell removal filter can be prepared by laminating polyester non-woven fabrics to form a laminated body or by winding such a polyester non-woven fabric into a cylindrical shape.

The term "surface-unmodified polyester non-woven fabric" of the present embodiment means that polyester is disposed on the surface of a non-woven fabric so that blood is directly contacted with the polyester, and also means that a hydrophilic treatment or the like is not performed on the surface of the polyester non-woven fabric.

An example of the hydrophilic treatment includes coating with a polymer.

It is preferable to use a polyester non-woven fabric having an effective mean fiber diameter as the polyester non-woven fabric of the present embodiment, since the fiber diameter of the polyester non-woven fabric contributes to adsorption or filtration capacity.

From viewpoints in that if the fiber diameter of the polyester non-woven fabric is too large, contact efficiency decreases, and if it is too small, the adsorbed amount of a clogging substance such as an aggregate increases, the mean fiber diameter of the polyester non-woven fabric is preferably within the range from 0.5 $\mu$m or more to 50 $\mu$m or less, more preferably within the range from 1 $\mu$m or more to 40 $\mu$m or less, and further preferably within the range from 1.5 $\mu$m or more to 35 $\mu$m or less.

The term "mean fiber diameter" of the present embodiment refers to a value measured by the following method.

Portions that are considered to be substantially uniform by visual observation are sampled from one or more sheets of polyester non-woven fabrics that form a cell removal filter.

Upon such sampling, an effective filtration cross section area portion, which corresponds to a portion through which blood passes during blood filtration in a polyester non-woven fabric, is divided into squares each having a side of 0.5 cm. From these squares, 6 portions are randomly sampled. Such random sampling is carried out, for example, by a method which comprises assigning addresses to all of the divided portions and then using a random number table, so as to select a necessary number of portions. Three or more, and preferably five or more each sampled portions are photographed at 2500-fold magnification using a scanning electron microscope.

A central region and regions close thereto are photographed for each sampled portion. Such regions are continuously photographed, until the total number of fibers photographed exceeds 100 for each sampled portion. Thereafter, the diameter of fibers is measured.

The diameter refers to a width of a fiber at right angle to the fiber axis. A sum of the diameters of all of the measured fibers is divided by the number of the fibers to obtain a mean fiber diameter.

However, in a case in which the widths of fibers cannot be measured because a plurality of fibers are overlapped and a fiber hides behind other fiber, in a case in which a plurality of fibers are melted to form a thick fiber, and in a case in which a fiber having a significantly different diameter is mixed, the widths of fibers are measured without adopting the data from these cases.

The phrase "a case in which the widths of fibers cannot be measured because a plurality of fibers are overlapped and a fiber hides behind other fiber" refers to a case in which there is a shadow in a scanning electron photomicrograph, and the phrase "a case in which a plurality of fibers are melted to form a thick fiber" refers to a case in which a fiber having a diameter 1.5-fold or more larger than the mean fiber diameter of other fibers is melted. The phrase "a case in which a fiber having a significantly different diameter is mixed" refers to a case in which a diameter is 2-fold or more larger than the mean fiber diameter of other fibers.

From the viewpoint of suppression of clogging, prevention of an increase in pressure loss, and smoothening of the flow, the weight per unit area of the polyester non-woven fabric is preferably from 20 g/cm$^2$ or more to 110 g/cm$^2$ or less and from 60 g/cm$^2$ or more to 100 g/cm$^2$ or less.

The term "filling density of the cell removal filter based on the vessel" of the present embodiment refers to the weight of the cell removal filter per constant volume when the cell removal filter is filled into the vessel. The filling density of the cell removal filter based on the vessel is preferably from 0.01 g/cm$^3$ or more to 0.5 g/cm$^3$ or less. From the viewpoint of an increase in the contact efficiency with a blood sample, suppression of clogging, prevention of an increase in pressure loss, and smoothening of the flow, the filling density is more preferably from 0.03 g/cm$^3$ or more to 0.3 g/cm$^3$ or less.

The cell removal filter of the present embodiment is filled with a filling solution comprising an antioxidant.

The term "antioxidant" of the present embodiment refers to an atom, a molecule or an ion having a property of easily donating an electron to other molecules and the like. The antioxidant has a property of suppressing the change of the cell removal filter caused by oxygen.

The antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.

An example of ascorbic acid derivatives includes, but is not particularly limited to, 5-methyl-3,4-dihydroxytetrone (MDT).

The antioxidant may be used in the form of either a single agent or a mixture of two or more types.

From the viewpoint of handling ability and safety, the antioxidant is preferably sodium pyrosulfite.

The phrase "the cell removal filter is filled with a filling solution" of the present embodiment refers to a state in which a filling solution is retained such that it is allowed to come into contact with the cell removal filter and thus, the cell removal filter is immersed in the filling solution.

The term "filling solution" of the present embodiment refers to a liquid filled in the selective removal device at the stage of production of the selective removal device, and an antioxidant is added to the filling solution.

The filling solution comprising an antioxidant can be prepared by directly adding an antioxidant to a filling solution that is water or a physiological solution such as a saline and dissolving it therein, or by previously dissolving an antioxidant in a small amount of solvent such as alcohol and then adding the obtained solution to a filling solution.

The concentration of an antioxidant in a solution comprising the same is determined to be an optimal concentration, depending on the type of an antioxidant and conditions for sterilization, which are applied to the selective removal device. The concentration of the antioxidant is preferably from 100 ppm or more to 1000 ppm or less, and more preferably from 300 ppm ro moreto 1000 ppm or less.

The term "radiation sterilization" of the present embodiment refers to the sterilization or removal of microorganisms existing in the cell removal filter filled with a filling solution.

The term "radiation" of the present embodiment refers to ionizing radiation including particle radiation such as alpha ray, beta ray, neutron ray or electron beam, and electromagnetic wave such as X ray or gamma ray. From the viewpoint of regular use in sterilization of medical devices, electron beam or gamma ray is preferable.

The electron beam is charged particle beam. The charged particle beam directly gives an electrical force to an orbital electron of the atom of a substance to which the beam is applied, or to an electron bound to a molecule, so as to cause ionization.

The gamma ray generates charged particle beam as a result of the interaction with the atom or atomic nucleus of a substance to which the beam is applied, and the thus secondarily generated charged particle beam has an ionization effect.

The effect of sterilizing microorganisms by electron beam or gamma ray of the present embodiment is obtained by the phenomenon whereby free radicals generated as a result of ionization damage the DNA strand of the microorganisms and inhibit their cell division capacity. Thus, the basic sterilization principle is the same.

The amount of radiation used to such radiation sterilization is different depending on the types of bacteria existing in the selective removal device, the number of bacteria, and an intended certified level of sterilization. Thus, the amount of radiation used can be individually determined by measuring a D value or carrying out validation. The amount of radiation used is preferably from 10 kGy or more to 100 kGy or less, which is generally applied, more preferably from 10 kGy or more to 50 kGy or less, and particularly preferably from 15 kGy or more to 40 kGy or less.

The term "D value" of the present embodiment refers to a sterilization processing unit (time, absorbed dose, etc.), at which 90% of specific microorganisms are killed, or the number of specific microorganisms is reduced to one-tenth.

When compared with gamma ray, the application of a certain dose of electron beam is completed in a shorter time. As a result, a dose applied per time (dose rate) is high, and the quantities of radicals generated are generally small. Thus, the electron beam tends to reduce the oxidative degradation of a substance to which the beam is applied. This is because oxygen is consumed on the surface of the substance if the dose rate is high, and thus, the oxygen is exhausted before it is diffused into the substance.

It is likely that the level of polyester decomposition is lower in the case of performing electron beam sterilization than in the case of performing gamma ray sterilization. However, it does not mean that no radicals are generated.

HLA-DR-positive monocytes can be selectively removed from blood comprising an anticoagulant by use of the device for selectively removing HLA-DR-positive monocytes of the present embodiment. By selectively removing HLA-DR-positive monocytes, the efficiency is considered to be improved in comparison with the therapeutic effect of the conventional leukocyte removal therapy.

The term "HLA-DR-positive cell" of the present embodiment refers to a blood cell that expresses an HLA-DR antigen on the surface thereof, among leukocytes that are human blood components.

The term "HLA-DR-positive monocyte" of the present embodiment refers to an HLA-DR-positive cell that is a monocyte, whereas the term "HLA-DR-positive B cell" of the present embodiment refers to an HLA-DR-positive cell that is a B cell.

The term "blood comprising an anticoagulant" of the present embodiment refers to fresh blood collected from a human, into which an anticoagulant is mixed.

The phrase "HLA-DR-positive monocytes can be selectively removed" of the present embodiment means that the percentage of the removed HLA-DR-positive monocytes based on the removed leukocytes is 12% or more.

The anticoagulant of the present embodiment is at least one selected from the group consisting of citric acid or a salt thereof.

Examples of an anticoagulant include, but are not particularly limited to, a sodium citrate solution, an ACD-A solution, and a CPD solution. The ACD-A (acid-citrate-dextrose) solution is a solution which consists of sodium citrate, citric acid and glucose, whereas the CPD (citrate-phosphate-dextrose) solution is a solution which consists of sodium citrate, citric acid, glucose, and sodium dihydrogen phosphate dehydrate.

The anticoagulant may be used in the form of either a single agent or a mixture of two or more types.

The device for selectively removing HLA-DR-positive monocytes of the present embodiment preferably comprises:

a cell removal filter filled with a filling solution comprising an antioxidant and subjected to radiation sterilization, wherein the cell removal filter is connected with a first flow passage on an upstream side and a second flow passage on a downstream side; and

a vessel containing at least one anticoagulant selected from the group consisting of citric acid and a salt thereof, wherein the vessel is connected with the first flow passage and is capable of supplying blood comprising the anticoagulant to the cell removal filter.

A selective removal device 10 has the configuration exemplified in Figure 1. A first flow passage 20 is a flow passage connecting to a vessel 30 containing an anticoagulant and a cell removal filter 40. The first flow passage 20 is connected to a liquid-supplying pump 50, and it is capable of supplying blood collected from a patient to the cell removal filter 40. The anticoagulant stored in the vessel 30 is supplied to the first flow passage 20, and it is mixed with blood that runs through the first flow passage 20. The blood comprising the anticoagulant is introduced into the cell removal filter 40, so that it can be filtrated. A second flow passage 60 is a flow passage in which the blood that has been filtered through the cell removal filter is led out of the filter.

The method for selectively removing HLA-DR-positive monocytes from blood comprising an anticoagulant of the present embodiment is a method in that HLA-DR-positive monocytes can be selectively removed from blood, by use of a selective removal device comprising a cell removal filter formed by a surface-unmodified polyester non-woven fabric; wherein the method comprises the following steps:

a step of filling the cell removal filter with a filling solution comprising an antioxidant;
a step of subjecting the cell removal filter filled with the filling solution to radiation sterilization; and
a step of supplying blood that comprises an anticoagulant comprising citric acid or a salt thereof to the filter device and filtrating it.

The first step of the present embodiment is a step of filling the cell removal filter with a filling solution by supplying the filling solution into the selective removal device.

The second step of the present embodiment is a step of subjecting the cell removal filter filled with the filling solution by the first step to radiation sterilization in the presence of the filling solution.

An Example of the method of carrying out radiation sterilization includes, but is not particularly limited to, a method of wrapping the device for selectively removing HLA-DR-positive monocytes and applying radiation thereto.

An Example of a radiation sterilization device includes, but is not particularly limited to, a JS9500-type radiation sterilization device (AECL).

The third step of the present embodiment is a step of supplying blood comprising an anticoagulant to the cell removal filter that has been subjected to radiation sterilization by the second step and filtrating it.

By filtrating the blood through the cell removal filter, HLA-DR-positive monocytes are captured by the cell removal filter, so that the HLA-DR-positive monocytes can be selectively removed.

In the present embodiment, it is preferable to previously wash the cell removal filter in order to remove the antioxidant from the filling solution before the supply of blood in the third step. As a solution used for washing, various types of physiological solutions can be used. A solution prepared by adding an anticoagulant to a physiological solution can be used to suppress stimulation to blood coagulation factors. Among others, a saline or a solution prepared by adding an anticoagulant to a saline can be preferably used.

Blood from which HLA-DR-positive monocytes have been selectively removed can be produced by use of the selective removal device of the present embodiment.

In the present embodiment, a method for producing blood from which HLA-DR-positive monocytes have been selectively removed comprises steps of:

filling a cell removal filter with a filling solution comprising an antioxidant;
subjecting the cell removal filter filled with the filling solution to radiation sterilization; and
supplying blood that comprises an anticoagulant comprising citric acid or a salt thereof to the filter device and filtrating it.

The above-mentioned filling solution comprising an antioxidant is preferably a filling solution comprising 300 ppm or more of sodium pyrosulfite in the use of an antioxidant for production of a device for selectively removing HLA-DR-positive monocytes of the present embodiment.

The above-mentioned radiation is preferably gamma ray or electron beam in the use of an antioxidant for production of a device for selectively removing HLA-DR-positive monocytes of the present embodiment.

In the present embodiment, the status of clinical use of the device for selectively removing HLA-DR-positive monocytes is not particularly limited, although there is the following case, for example.

In a case in which the selective removal device is used clinically for a patient with rheumatoid arthritis or ulcerative colitis, it is preferable to treat 3 L of blood with the device for selectively removing HLA-DR-positive monocytes once a week, repeatedly for a total of 5 times.

In the present embodiment, the treatment of 3 L of blood means as follows.

First, 1 L of a physiological solution connected with a blood collection circuit is supplied to the device for selectively removing HLA-DR-positive monocytes, which is connected with the blood collection circuit, a liquid-supplying pump and a blood retransfusion circuit. Subsequently, 500 mL of a physiological solution to which an anticoagulant has been added is supplied to the device for selectively removing HLA-DR-positive monocytes.

Thereafter, the blood collection circuit is connected with the cubital vein or the like of a patient, and the blood retransfusion circuit is connected with the other cubital vein or the like of the patient. A conduit that leads to the physiological solution comprising the anticoagulant is connected at the branch of the blood collection circuit.

The liquid-supplying pump is operated at 50 mL/min of a flow rate for 1 hour, and 3 L of blood to which the physiological solution comprising the anticoagulant has been added is supplied to the selective removal device. After the supply of 3 L of blood has been completed, a physiological solution is connected with the blood collection circuit, and 500 mL of a physiological solution is supplied to the selective removal device, so that the blood is returned to the patient.

A method of adding a physiological solution comprising an anticoagulant to blood is not particularly limited. For example, there is a method of adding a physiological solution comprising 4% (w/v) sodium citrate to blood by operating a pump such that 1 volume of the physiological solution is used based on 5 to 40 volumes of blood.

The blood collection circuit consists of a blood introduction unit capable of being connected with cubital vein or the like and a conduit that connects the blood introduction unit with the selective removal device. The blood collection circuit has a branch to a conduit that leads to a physiological solution comprising an anticoagulant. A liquid-supplying pump that can be controlled separately from the blood collection circuit is connected in the conduit that leads to a physiological solution comprising an anticoagulant,.

The term "blood introduction unit" of the present embodiment refers to a portion for introducing blood to the conduit.

Examples of a blood introduction unit include, but are not particularly limited to, a spike needle and a blood collection needle.

Any type of conduit may be used, as long as it is able to supply blood, a physiological solution and the like and to carry out solution sending in safety for the living body. In addition, any type of the material of the conduit may be used, as long as it is a material, from which harmful substances are not eluted with a solution such as blood and a physiological solution.

The type of the conduit is not particularly limited. Examples of the conduit include, but are not particularly limited to, vinyl chloride, silicon, polypropylene, and polyethylene. From the viewpoint of strength, vinyl chloride is preferably used as the conduit. A specific example of vinyl chloride includes LC-3000N (Asahi Kasei Kuraray Medical CO., LTD.).

The liquid-supplying pump may be a liquid-supplying means. For example, all types of known means using a pump or the like may be used. Examples of the pump preferably used herein include a roller pump and a finger pump. A pump capable of supplying a liquid at a flow rate of 20 mL/min to 300 mL/min with high precision is preferable. A specific example of such a pump includes Plasauto LC (Asahi Kasei Kuraray Medical CO., LTD.).

The term "physiological solution" of the present embodiment refers to a solution almost isotonic with a body fluid, wherein the solution is prepared, such that living cells or tissues exhibit normal physiological functions as much as possible therein.

Examples of the physiological solution include, but are not particularly limited to, physiological salt solutions such as a physiological saline solution (including a saline), a Ringer's solution, a lactate Ringer's solution, an acetate Ringer's solution or a Tyrode solution; sugar-containing solutions such as glucose, dextran or heparin; and anticoagulant-containing solutions.

A solution prepared by diluting a mixed solution comprising at least one of the above-exemplified physiological salt solutions, sugar-containing solutions, anticoagulant-containing solutions and the like is also preferably used as the physiological solution,

The physiological solution may be used in the form of either a single solution or a mixture of two or more types. Among others, a physiological saline solution is used.

In the present embodiment, a blood preparation, from which HLA-DR-positive monocytes are selectively removed, can be obtained by treating blood collected from a patient using a device for selectively removing HLA-DR-positive monocytes.

Examples of the blood collected from a patient include, but are not particularly limited to, whole blood, plasma, and a blood-derived preparation such as an erythrocyte preparation and a platelet preparation.

Examples

Hereinafter, the present embodiment will be more specifically described in examples and comparative examples. However, these examples are not intended to limit the present embodiment.

[Example 1]

A polyethylene terephthalate (PET) non-woven fabric (mean fiber diameter: 2.3 $\mu$m; weight per unit area: 90 g/m$^2$; thickness: 0.45 mm) was cut into round pieces each having a diameter of 6.8 mm, and 11 pieces were piled on one another to produce a cell removal filter. The cell removal filter was laminated on the inside of a 1-mL column having a diameter of 6.8 mm made of polyethylene (Mobicol (registered trademark), MoBiTec) (filling density: 0.14 g/cm$^3$). The inside of the column was filled with an aqueous sodium pyrosulfite solution (sodium pyrosulfite; Hayashi Pure Chemical Ind., Ltd.), and the column was then placed in a cardboard case and wrapped. Then, a sterilization treatment was carried out by radiation of 25 kGy of gamma ray. The obtained column was defined as a selective removal device. The concentration of the sodium pyrosulfite in the aqueous solution was set at 800 ppm.

Blood was collected from a healthy volunteer, and a sodium citrate solution (Citramin (registered trademark), Fuso Pharmaceutical Industries, Ltd.) containing 4% (w/v) of sodium citrate hydrate as an anticoagulant was added to the blood, such that the obtained mixture contained 9.1% (v/v) of the sodium citrate solution to prepare fresh human blood with the sodium citrate solution (the final concentration of the sodium citrate hydrate: 0.36 g/100 mL) as a pre-filtration sample.

The sterilized selective removal device was washed by supplying a saline (Otsuka Normal Saline (Otsuka Seisyokuchuu) (registered trademark), Otsuka pharmaceutical Co., Ltd.) thereto, and the pre-filtration sample was then filtered through the device at a rate of 0.18 mL/min for 10 minutes. Blood discharged from the outlet during the operation was used as a post-filtration sample in the subsequent measurement.

The leukocyte removal rate was calculated according to the following formula (1) by measuring leukocyte concentrations before and after filtration using an automated blood cell counter (Sysmex SF-3000 (manufactured by Toa Iyo Denshi Co., Ltd.)).

Formula (1) :

$$\texttt{Leukocyte removal rate (\%) = [1-(leukocyte concentration after filtration/leukocyte concentration before filtration)]} \times 100$$

[Comparative Example 1]

A leukocyte removal device was obtained in the similar method as Example 1 with the exception that water (containing no sodium pyrosulfite) was used as a filling solution.

A 10 mg/100 mL nafamostat mesilate solution (Futhan (registered trademark), Trii Pharmaceutical Co., Ltd.; the same as below) as an anticoagulant was added such that the obtained mixture contained 12% (v/v) of the nafamostat mesilate solution to prepare fresh human blood with the nafamostat mesilate solution (the final concentration of the nafamostat mesilate: 1.2 mg/100 mL) as a pre-filtration sample. Using the above described leukocyte removal device, the pre-filtration sample was filtrated in the similar method as Example 1. The leukocyte removal rate was calculated by measuring leukocyte concentrations.

[Comparative Example 2]

A 10 mg/100 mL nafamostat mesilate solution as an anticoagulant was added such that the obtained mixture contained 12% (v/v) of the nafamostat mesilate solution to prepare fresh human blood with addition of the nafamostat mesilate solution (the final concentration of the nafamostat mesilate: 1.2 mg/100 mL) as a pre-filtration sample. Using the selective removal device of Example 1, the pre-filtration sample was filtrated in the similar method as Example 1. The leukocyte removal rate was calculated by measuring leukocyte concentrations.

[Comparative Example 3]

Filtration was carried out in the similar method as Example 1 with the exception that the leukocyte removal device produced in the similar manner as Comparative Example 1. The leukocyte removal rate was calculated by measuring leukocyte concentrations.

[Examples 2 to 4]

The leukocyte removal rate was calculated in the similar method as Example 1 with the exception that the column produced in the similar manner as Example 1 was filled with 100 ppm, 300 ppm or 1000 ppm of an aqueous sodium pyrosulfite solution.

[Example 5]

An ACD-A solution (containing 0.8% citric acid, 2.2% sodium citrate and 2.2% glucose solution; Terumo Corporation) as an anticoagulant was added such that the obtained mixture contained 12% (v/v) of the ACD-A solution to prepare fresh human blood with the ACD-A solution as a pre-filtration sample. Using the selective removal device of Example 1, the pre-filtration sample was filtrated in the similar method as Example 1. The leukocyte removal rate was calculated by measuring leukocyte concentrations.

The results of Examples 1 to 5 and Comparative Examples 1 to 3 are shown in Table 1.

The method for testing the selective removal of HLA-DR-positive monocytes, used in the present embodiment, is as follows.

[Test Example 1]

Each of the pre-filtration and post-filtration samples was reacted with an anti-HLA-DR antibody (BD Biosciences), which had been fluorescently labeled and then analyzed by flow cytometry (FACS Calibur; BD Biosciences). Those that had been reacted with the HLA-DR antibody were defined as HLA-DR-positive cells, and the concentration of the HLA-DR-positive cells in each sample was calculated.

The proportions of the removed HLA-DR-positive cells based on leukocytes in each of examples and comparative examples were calculated according to the following formula (2).

Formula (2):

```
The proportion (%) of the removed HLA-DR-positive
cells based on leukocytes = (HLA-DR-positive cell
concentration before filtration - HLA-DR-positive cell
concentration after filtration) / (leukocyte
concentration before filtration - leukocyte concentration
after filtration) × 100
```

The proportions of the removed HLA-DR-positive cells based on leukocytes were found to be 20.8%, 17.2%, 19.5%, 26.9% and 15.7% in Examples 1 to 5, respectively. In addition, in Comparative Examples 1 to 3, the proportions of the removed HLA-DR-positive cells based on leukocytes were found to be 7.5%, 7.5% and 16.0%, respectively.

The proportions were calculated by setting the value of Comparative Example 1 at 1.0, and the results are shown in Table 1.

[Table 1]

| | Example 1 | Example 2 | Example 3 | Example 4 | Example 5 |
|---|---|---|---|---|---|
| Concentration of sodium pyrosulfite in filling solution (ppm) | 800 | 100 | 300 | 1000 | 800 |
| Sterilization method | Gamma ray | Gamma ray | Gamma ray | Gamma ray | Gamma ray |
| Anticoagulant | Sodium citrate | Sodium citrate | Sodium citrate | Sodium citrate | Citric acid and sodium citrate |
| Proportion of removed HLA-DR-positive cells based on leukocytes (proportion) | 2.8 | 2.3 | 2.6 | 3.6 | 2.1 |
| Leukocyte removal rate (%) | 77.4 | 83.2 | 79.4 | 67.6 | 77.4 |

| | Comparative Example 1 | Comparative Example 2 | Comparative Example 3 |
|---|---|---|---|
| Concentration of sodium pyrosulfite in filling solution (ppm) | 0 | 800 | 0 |
| Sterilization method | Gamma ray | Gamma ray | Gamma ray |
| Anticoagulant | Nafamostat mesilate | Nafamostat mesilate | Sodium citrate |
| Proportion of removed HLA-DR-positive cells based on leukocytes (proportion) | 1 | 1 | 2.1 |
| Leukocyte removal rate (%) | 99.4 | 99.7 | 85.1 |

EP 2 361 644 B1

[Test Example 2]

Each of the samples were reacted with an anti-HLA-DR antibody and an anti-CD14 antibody (BD Biosciences), which had been fluorescently labeled and then analyzed by flow cytometry in the similar manner as Test Example 1. Those that had been reacted with the anti-HLA-DR antibody and the anti-CD14 antibody were defined as HLA-DR-positive monocytes, and the concentration of the HLA-DR-positive monocytes in each sample was calculated.

The proportions of the removed HLA-DR-positive monocytes based on leukocytes in each of examples and comparative examples were calculated according to the following formula (3).

Formula (3):

$$
\begin{aligned}
&\text{The proportion (\%) of the removed HLA-DR-positive} \\
&\text{monocytes based on leukocytes = (HLA-DR-positive monocyte} \\
&\text{concentration before filtration - HLA-DR-positive} \\
&\text{monocyte concentration after filtration) / (leukocyte} \\
&\text{concentration before filtration - leukocyte concentration} \\
&\text{after filtration)} \times 100
\end{aligned}
$$

The proportions of the removed HLA-DR-positive monocytes based on leukocytes were found to be 18.3%, 12.2%, 16.5%, 28.4% and 18.6% in Examples 1 to 5, respectively. In addition, in Comparative Examples 1 to 3, the proportions of the removed HLA-DR-positive monocytes based on leukocytes were found to be 8.7%, 8.7% and 10.3%, respectively.

The proportions were calculated by setting the value of Comparative Example 1 at 1.0, and the results are shown in Table 2.

[Table 2]

|  | Example 1 | Example 2 | Example 3 | Example 4 | Example 5 |
|---|---|---|---|---|---|
| Concentration of sodium pyrosulfite in filling solution (ppm) | 800 | 100 | 300 | 1000 | 800 |
| Sterilization method | Gamma ray | Gamma ray | Gamma ray | Gamma ray | Gamma ray |
| Anticoagulant | Sodium citrate | Sodium citrate | Sodium citrate | Sodium citrate | Citric acid and sodium citrate |
| Proportion of removed HLA-DR-positive monocytes based on leukocytes (proportion) | 2.1 | 1.4 | 1.9 | 3.3 | 2.1 |

|  | Comparative Example 1 | Comparative Example 2 | Comparative Example 3 |
|---|---|---|---|
| Concentration of sodium pyrosulfite in filling solution (ppm) | 0 | 800 | 0 |
| Sterilization method | Gamma ray | Gamma ray | Gamma ray |
| Anticoagulant | Nafamostat mesilate | Nafamostat mesilate | Sodium citrate |
| Proportion of removed HLA-DR-positive monocytes based on leukocytes (proportion) | 1 | 1 | 1.2 |

EP 2 361 644 B1

[Test Example 3]

Each of the samples were reacted with an anti-HLA-DR antibody and an anti-CD19 antibody (BD Biosciences), which had been fluorescently labeled and then analyzed by flow cytometry in the similar manner as Test Example 1. Those that had been reacted with the anti-HLA-DR antibody and the anti-CD19 antibody were defined as HLA-DR-positive B cells, and the concentration of the HLA-DR-positive B cells in each sample was calculated.

The proportions of the removed HLA-DR-positive B cells based on leukocytes in each of examples and comparative examples were calculated according to the following formula (4).

Formula (4):

$$\text{The proportion (\%) of the removed HLA-DR-positive B cells based on leukocytes} = (\text{HLA-DR-positive B cell concentration before filtration} - \text{HLA-DR-positive B cell concentration after filtration}) / (\text{leukocyte concentration before filtration} - \text{leukocyte concentration after filtration}) \times 100$$

The proportions of the removed HLA-DR-positive B cells based on leukocytes were found to be 24.6%, 24.6%, 24.6%, 24.6% and 24.8% in Examples 1 to 5, respectively. In addition, in Comparative Examples 1 to 3, the proportions of the removed HLA-DR-positive B cells based on leukocytes were found to be 5.6%, 5.6% and 24.6%, respectively.

The proportion of each removed fraction based on leukocytes was calculated by setting the value of Comparative Example 1 at 1.0, and the results are shown in Table 3.

[Table 3]

| | Example 1 | Example 2 | Example 3 | Example 4 | Example 5 |
|---|---|---|---|---|---|
| Concentration of sodium pyrosulfite in filling solution (ppm) | 800 | 100 | 300 | 1000 | 800 |
| Sterilization method | Gamma ray | Gamma ray | Gamma ray | Gamma ray | Gamma ray |
| Anticoagulant | Sodium citrate | Sodium citrate | Sodium citrate | Sodium citrate | Citric acid and sodium citrate |
| Proportion of removed HLA-DR-positive B cells based on leukocytes (proportion) | 4.4 | 4.4 | 4.4 | 4.4 | 4.4 |

| | Comparative Example 1 | Comparative Example 2 | Comparative Example 3 |
|---|---|---|---|
| Concentration of sodium pyrosulfite in filling solution (ppm) | 0 | 800 | 0 |
| Sterilization method | Gamma ray | Gamma ray | Gamma ray |
| Anticoagulant | Nafamostat mesilate | Nafamostat mesilate | Sodium citrate |
| Proportion of removed HLA-DR-positive B cells based on leukocytes (proportion) | 1 | 1 | 4.4 |

From the results shown in Tables 1 to 3, it is found that HLA-DR-positive monocytes were selectively removed in Examples 1 to 5, in which a selective removal device was sterilized by gamma ray in the presence of a filling solution containing sodium pyrosulfite, and blood was then treated in the presence of an anticoagulant containing citric acid and/or sodium citrate.

It is assumed that the selective removal of HLA-DR-positive monocytes would be involved in a decrease in the adhesive capacity of cells due to the calcium chelating action of citric acid and/or a salt thereof.

Moreover, it is considered that, as a result of a decrease in negative charge on the surface of a non-woven fabric due to the antioxidant action of the filling solution, selectivity to HLA-DR-positive monocytes was increased.

The present application is based on a Japanese patent application (Japanese Patent Application No. 2008-300176), filed on November 25, 2008; the disclosure of which is hereby incorporated by reference.

Industrial Applicability

The device for selectively removing HLA-DR-positive monocytes of the present invention can be effectively used to selectively remove HLA-DR-positive monocytes.

The use of an antioxidant for production of the device for selectively removing HLA-DR-positive monocytes, device for selectively removing HLA-DR-positive monocytes, and method for selectively removing HLA-DR-positive monocytes of the present invention have industrial applicability in the field of treatment of autoimmune diseases such as systemic lupus erythematosus, rheumatoid arthritis (including malignant rheumatoid arthritis), multiple sclerosis, ulcerative colitis and Crohn's disease, leukemia, and other diseases.

Reference Signs List

10    Selective removal device
20    Fist flow passage
30    Vessel containing anticoagulant
40    Cell removal filter
50    Liquid-supplying pump
60    Second flow passage

**Claims**

1. Use of an antioxidant for manufacture of a device (10) for selectively removing HLA-DR-positive monocytes, which selectively removes HLA-DR-positive monocytes from blood comprising an anticoagulant, wherein
the device (10) for selectively removing HLA-DR-positive monocytes comprises a cell removal filter (40) formed by a surface-unmodified polyester non-woven fabric, wherein the polyester is disposed on the surface of the non-woven fabric so that the blood is directly contacted with the polyester and wherein no hydrophilic treatment is performed on the surface of the polyester non-woven fabric,
the cell removal filter (40) is filled with a filling solution comprising the antioxidant and is subjected to radiation sterilization,
the anticoagulant is at least one selected from the group consisting of citric acid and a salt thereof, and
the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.

2. The use of the antioxidant according to claim 1, wherein the filling solution comprises 300 ppm or more of sodium pyrosulfite.

3. The use of the antioxidant according to claim 1 or 2, wherein the radiation is gamma ray or electron beam.

4. A device (10) for selectively removing HLA-DR-positive monocytes, which selectively removes HLA-DR-positive monocytes from blood comprising an anticoagulant, wherein
the device for selectively removing HLA-DR-positive monocytes comprises a cell removal filter (40) formed by a surface-unmodified polyester non-woven fabric, wherein the polyester is disposed on the surface of the non-woven fabric so that the blood is directly contacted with the polyester and wherein no hydrophilic treatment is performed on the surface of the polyester non-woven fabric,

the cell removal filter (40) is filled with a filling solution comprising an antioxidant and is subjected to radiation sterilization,

the anticoagulant is at least one selected from the group consisting of citric acid and a salt thereof, and

the antioxidant is at least one selected from the group consisting of sodium pyrosulfite, potassium pyrosulfite, sodium thiosulfate, sodium hydrogen sulfite, sodium sulfite, hypophosphorous acid, ascorbic acid, and an ascorbic acid derivative.

5. The selective removal device (10) according to claim 4, wherein the filling solution comprises 300 ppm or more of sodium pyrosulfite.

6. The selective removal device (10) according to claim 4 or 5, wherein the radiation is gamma ray or electron beam.

7. The device (10) according to claim 4,

wherein the cell removal filter(40) is connected with a first flow passage (20) on an upstream side and a second flow passage (60) on a downstream side; and

a vessel (30) containing at least one anticoagulant selected from the group consisting of citric acid and a salt thereof, wherein the vessel (30) is connected with the first flow passage (20) and is capable of supplying blood comprising the anticoagulant to the cell removal filter(40).

**Patentansprüche**

1. Verwendung eines Antioxidans zur Herstellung einer Vorrichtung (10) zum selektiven Entfernen von HLA-DR-positiven Monocyten, die selektiv HLA-DR-positive Monocyten aus Blut, das ein Antikoagulans umfasst, entfernt, wobei die Vorrichtung (10) zum selektiven Entfernen von HLA-DR-positiven Monocyten einen Zellentfernungsfilter (40) umfasst, der aus einem Vliesstoff aus nicht oberflächenmodifiziertem Polyester gebildet ist, wobei sich der Polyester auf der Oberfläche des Vliesstoffs befindet, so dass das Blut direkt mit dem Polyester in Kontakt kommt und wobei keine hydrophile Behandlung auf der Oberfläche des Polyester-Vliesstoffs durchgeführt wird,

der Zellentfernungsfilter (40) mit einer Fülllösung, die das Antioxidans umfasst, gefüllt ist und einer Strahlungssterilisation unterzogen wird;

das Antikoagulans wenigstens eines ist, das aus der Gruppe ausgewählt ist, die aus Zitronensäure und einem Salz davon besteht; und

das Antioxidans wenigstens eines ist, das aus der Gruppe ausgewählt ist, die aus Natriumpyrosulfit, Kaliumpyrosulfit, Natriumthiosulfat, Natriumhydrogensulfit, Natriumsulfit, hypophosphoriger Säure, Ascorbinsäure und einem Ascorbinsäurederivat besteht.

2. Verwendung des Antioxidans gemäß Anspruch 1, wobei die Fülllösung 300 ppm oder mehr Natriumpyrosulfit umfasst.

3. Verwendung des Antioxidans gemäß Anspruch 1 oder 2, wobei es sich bei der Strahlung um Gammastrahlen oder Elektronenstrahlen handelt.

4. Vorrichtung (10) zum selektiven Entfernen von HLA-DR-positiven Monocyten, die selektiv HLA-DR-positive Monocyten aus Blut, das ein Antikoagulans umfasst, entfernt, wobei

die Vorrichtung (10) zum selektiven Entfernen von HLA-DR-positiven Monocyten einen Zellentfernungsfilter (40) umfasst, der aus einem Vliesstoff aus nicht oberflächenmodifiziertem Polyester gebildet ist, wobei sich der Polyester auf der Oberfläche des Vliesstoffs befindet, so dass das Blut direkt mit dem Polyester in Kontakt kommt und wobei keine hydrophile Behandlung auf der Oberfläche des Polyester-Vliesstoffs durchgeführt wird,

der Zellentfernungsfilter (40) mit einer Fülllösung, die das Antioxidans umfasst, gefüllt ist und einer Strahlungssterilisation unterzogen wird;

das Antikoagulans wenigstens eines ist, das aus der Gruppe ausgewählt ist, die aus Zitronensäure und einem Salz davon besteht; und

das Antioxidans wenigstens eines ist, das aus der Gruppe ausgewählt ist, die aus Natriumpyrosulfit, Kaliumpyrosulfit, Natriumthiosulfat, Natriumhydrogensulfit, Natriumsulfit, hypophosphoriger Säure, Ascorbinsäure und einem Ascorbinsäurederivat besteht.

5. Vorrichtung (10) zum selektiven Entfernen gemäß Anspruch 4, wobei die Fülllösung 300 ppm oder mehr Natriumpyrosulfit umfasst.

**6.** Vorrichtung (10) zum selektiven Entfernen gemäß Anspruch 4 oder 5, wobei es sich bei der Strahlung um Gammastrahlen oder Elektronenstrahlen handelt.

**7.** Vorrichtung (10) gemäß Anspruch 4,
wobei der Zellentfernungsfilter (40) mit einem ersten Strömungsdurchlass (20) auf einer Stromaufwärts-Seite und einem zweiten Strömungsdurchlass (60) auf einer Stromabwärts-Seite verbunden ist; und
ein Gefäß (30), das wenigstens ein Antikoagulans enthält, welches aus der Gruppe ausgewählt ist, die aus Zitronensäure und einem Salz davon besteht, wobei das Gefäß (30) mit dem ersten Strömungsdurchlass (20) verbunden ist und Blut, das das Antikoagulans umfasst, dem Zellentfernungsfilter (40) zuführen kann.

**Revendications**

**1.** Utilisation d'un antioxydant pour la fabrication d'un dispositif (10) permettant d'éliminer sélectivement des monocytes positifs à HLA-DR, qui élimine sélectivement des monocytes positifs à HLA-DR du sang comprenant un anticoagulant, dans laquelle
le dispositif (10) permettant d'éliminer sélectivement des monocytes positifs à HLA-DR comprend un filtre d'élimination de cellules (40) formé par une étoffe non tissée en poly(ester) non modifiée en surface, dans lequel le poly(ester) est disposé sur la surface de l'étoffe non tissée de sorte que le sang est directement mis en contact avec le poly(ester) et dans lequel aucun traitement hydrophile n'est réalisé sur la surface de l'étoffe non tissée en poly(ester),
le filtre d'élimination de cellules (40) est rempli d'une solution de remplissage comprenant l'antioxydant et est soumis à une stérilisation par rayonnement,
l'anticoagulant est au moins un élément choisi dans le groupe consistant en l'acide citrique et un sel de celui-ci, et
l'antioxydant est au moins un élément choisi dans le groupe consistant en le pyrosulfite de sodium, le pyrosulfite de potassium, le thiosulfate de sodium, l'hydrogénosulfite de sodium, le sulfite de sodium, l'acide hypophosphoreux, l'acide ascorbique et un dérivé d'acide ascorbique.

**2.** Utilisation de l'antioxydant selon la revendication 1, dans laquelle la solution de remplissage comprend 300 ppm ou plus de pyrosulfite de sodium.

**3.** Utilisation de l'antioxydant selon la revendication 1 ou 2, dans laquelle le rayonnement est un rayon gamma ou un faisceau d'électrons.

**4.** Dispositif (10) permettant d'éliminer sélectivement des monocytes positifs à HLA-DR, qui élimine sélectivement des monocytes positifs à HLA-DR de sang comprenant un anticoagulant, dans lequel
le dispositif (10) permettant d'éliminer sélectivement des monocytes positifs à HLA-DR comprend un filtre d'élimination de cellules (40) formé par une étoffe non tissée en poly(ester) non modifiée en surface, dans lequel le poly(ester) est disposé sur la surface de l'étoffe non tissée de sorte que le sang est directement mis en contact avec le poly(ester) et dans lequel aucun traitement hydrophile n'est réalisé sur la surface de l'étoffe non tissée en poly(ester),
le filtre d'élimination de cellules (40) est rempli d'une solution de remplissage comprenant un antioxydant et est soumis à une stérilisation par rayonnement,
l'anticoagulant est au moins un élément choisi dans le groupe consistant en l'acide citrique et un sel de celui-ci, et
l'antioxydant est au moins un élément choisi dans le groupe consistant en le pyrosulfite de sodium, le pyrosulfite de potassium, le thiosulfate de sodium, l'hydrogénosulfite de sodium, le sulfite de sodium, l'acide hypophosphoreux, l'acide ascorbique et un dérivé d'acide ascorbique.

**5.** Dispositif d'élimination sélectif (10) selon la revendication 4, dans lequel la solution de remplissage comprend 300 ppm ou plus de pyrosulfite de sodium.

**6.** Dispositif d'élimination sélectif (10) selon la revendication 4 ou 5, dans lequel le rayonnement est un rayon gamma ou un faisceau d'électrons.

**7.** Dispositif (10) selon la revendication 4,
dans lequel le filtre d'élimination de cellules (40) est raccordé à un premier passage d'écoulement (20) sur un côté amont et un second passage d'écoulement (60) sur un côté aval ; et

une cuve (30) contenant au moins un anticoagulant choisi dans le groupe consistant en l'acide citrique et un sel de celui-ci, dans lequel la cuve (30) est raccordée au premier passage d'écoulement (20) et est capable de fournir du sang comprenant l'anticoagulant au filtre d'élimination de cellules (40).

Figure 1

30

10

20

patient

50

40

60

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11322618 A
- JP 3812909 B
- EP 1553113 A1
- JP 2008300176 A

**Non-patent literature cited in the description**

- **MOTTONEN M.** *Immunology,* 2000, vol. 100, 238-244
- **SAWADA K.** *Apheresis,* 1995, vol. 14 (1), 48-50